Europäisches Patentamt

European Patent Office

Office européen des brevets

(11) Publication number: **0 577 932 A2**

# EUROPEAN PATENT APPLICATION

(21) Application number: 93105720.2

(22) Date of filing: 07.04.93

(51) Int. Cl.5: **C12Q 1/68**, G01N 33/74

---

The applicant has subsequently filed a sequence listing and declared, that it includes no new matter.

(30) Priority: **08.05.92 US 881526**

(43) Date of publication of application:
**12.01.94 Bulletin 94/02**

(84) Designated Contracting States:
**AT BE CH DE DK ES FR GB GR IE IT LI LU NL PT SE**

(71) Applicant: **AMERICAN CYANAMID COMPANY**
**1937 West Main Street**
**P.O. Box 60**
**Stamford Connecticut 06904-0060(US)**

(72) Inventor: **Mak, Paul**
**18 Hankins Road**
**East Windsor, New Jersey 08520(US)**

(74) Representative: **Wächtershäuser, Günter, Dr.**
**Tal 29**
**D-80331 München (DE)**

---

(54) **Development of a mechanism-based screen to identify androgen receptor agonists and antagonists.**

(57) The present invention relates to a novel androgen receptor screening method using a yeast-based expression system. The use of this yeast system enhances the ability to screen for agonists and/or antagonists of the steroid thyroid receptor superfamily. Furthermore, the use of this yeast system allows the localization and identification of functional domains of the androgen receptor.

EP 0 577 932 A2

## BACKGROUND OF THE INVENTION

The present invention relates to a novel androgen receptor screening method using a yeast-based expression system. The use of this yeast system enhances the ability to screen for agonists and/or antagonists of the Steroid/thyroid receptor Superfamily. Furthermore, the use of this yeast system allows the localisation and identification of functional domains of the androgen receptor.

In addition, the present invention relates to mouse androgen receptor mutants which are useful in determining the transcriptional activation domains of the androgen receptor.

Androgens play an important role in sexual differentiation, regulation of body composition and maintenance of normal male reproductive function. The mechanism of androgen action has been studied extensively since the discovery of the androgen receptors (1). Molecular cloning of the human and the rat androgen receptors (2,3,4,5) has revealed that these receptor proteins belong to the steroid and thyroid receptor superfamily (6,7). These steroid hormone receptors are ligand-activated transcription factors, which bind to hormone response elements at the promoter regions of target genes and subsequently regulate gene transcription (8,9).

The sequence of the mouse androgen receptor cDNA used in the present invention is similar to that reported by others (10,11). Comparison of amino acid sequences of mouse, rat and human androgen receptors indicate that the putative DNA- and ligand-binding domains are similar. However, the homology in the amino-terminal regions between mouse and human androgen receptors is only 74%. It has been shown by deletion analysis that the N-terminal region of the human androgen receptor contains a signal sequence essential for target gene activation. Although not verified as of yet, it has been observed that patients suffering from spinal and bulbar muscular atrophy (Kennedy's disease) have an extra long tract of glutamine residues near the amino-terminus of the androgen receptor gene (12). Thus, abnormality in this gene may be indeed be associated with certain diseases. Analogously, the mouse androgen receptor also contains a similar stretch of glutamine residues, the actual function of the amino-terminus of the receptor has not been analyzed. However, the present invention provides a convenient model system in which to study such functions.

In the past, mammalian vitamin $D_3$ receptors have been shown to function in yeast cells (13). Unlike those systems, the present invention clearly demonstrates functional expression of the mouse androgen receptor in yeast cells. Using a ubiquitin-fusion system and a protease-deficient yeast strain, the intact wild type and mutant receptor proteins, as shown by Western blot analyses, are expressed. The molecular masses of these expressed receptors correlate well with predicted sizes further supporting that the fusion protein is rapidly cleaved by yeast ubiquitinases within those host (13), thereby providing an efficient mechanism by which to screen for drugs and/or to study functional domains of the region.

## BRIEF DESCRIPTION OF THE DRAWINGS

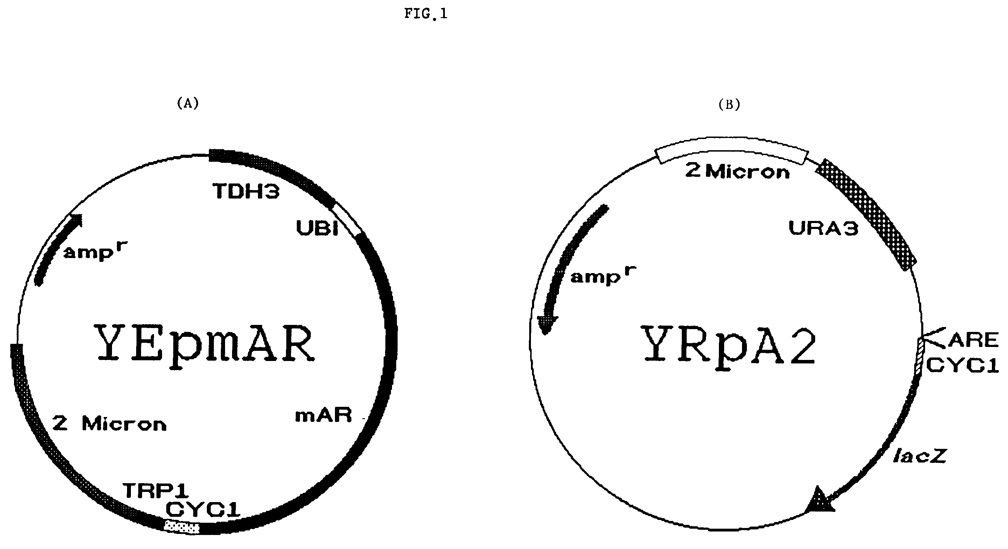

### FIGURE 1: (A) Yeast Expression Vector (YEpmAR)

The mouse androgen receptor (mAR) cDNA is inserted into the expression plasmid to produce an inframe fusion protein with ubiquitin (UBI) under the control of the yeast triose phosphate dehydrogenase (TDH3) promoter. TRP1 is the tryptophan selectable marker. 2 Micron is the yeast replicating DNA and CYC1 is the transcriptional terminator derived from the iso-1-cytochrome c gene.

### (B) Yeast Reporter Plasmid (YRpA2)

The yeast upstream regulatory elements of the iso-1-cytochrome c (CYC1) promoter are replaced by an oligonucleotide containing two copies of a putative androgen response element (ARE) inserted into the XhoI site upstream (-250 base pairs) of the CYC1 promoter which is fused to the lac-Zgene of E. coli. URA3 is the selectable marker.

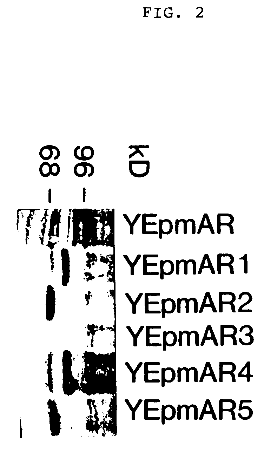

### FIGURE 2: Western Immunoblot Analysis Of Wild Type Mouse Androgen Receptor And Androgen Receptor Deletion Mutants Expressed In Yeast Cells

Yeast extracts (100 µg protein) containing the wild type (YEpmAR) or the receptor mutants (YEpmAR1, YEpmAR2, YEpmAR3, YEpmAR4 and YEpmAR5) are analyzed by Western blots as described in the examples. A polyclonal antibody directed against the N-terminus of the human androgen receptor is used to probe the mouse androgen receptors. Molecular weight markers are indicated in kilodalton (kDa).

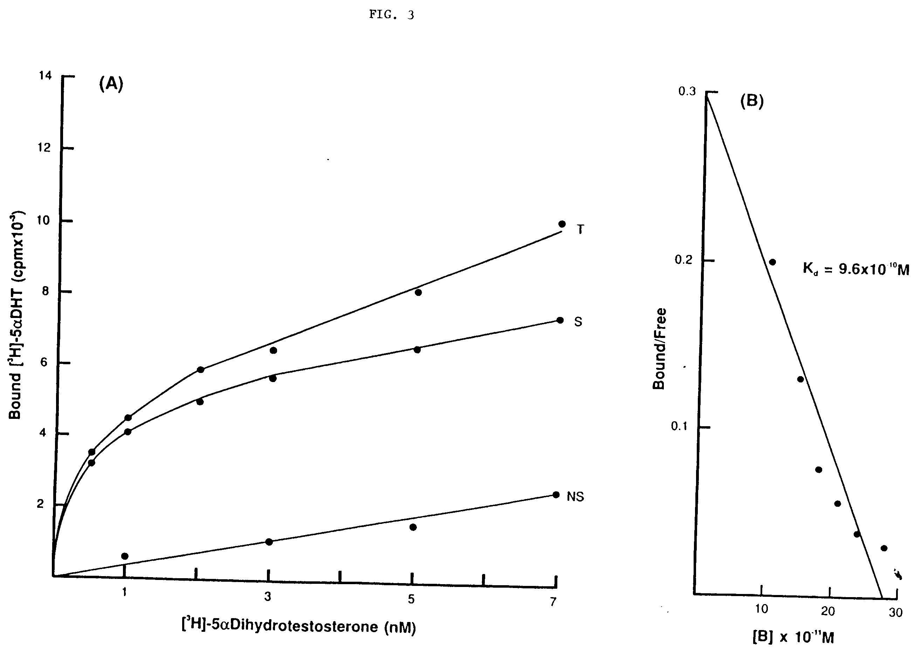

**FIGURE 3: Saturation Analysis Of Mouse Androgen Receptors Produced By Yeast Cells**

Aliquots of yeast extracts containing the wild type mouse androgen receptors are incubated with increasing concentrations of [$^3$H]5$\alpha$-Dihydrotestosterone in the absence (total binding, T) or presence (non-specific binding, NS) of a 100-fold molar excess of radioinert 5$\alpha$-Dihydrotestosterone. Specific binding (S) is determined by subtracting non-specific from total binding. (B) Scatchard analysis of the data from (A), two other separate experiments, give similar apparent Kd values.

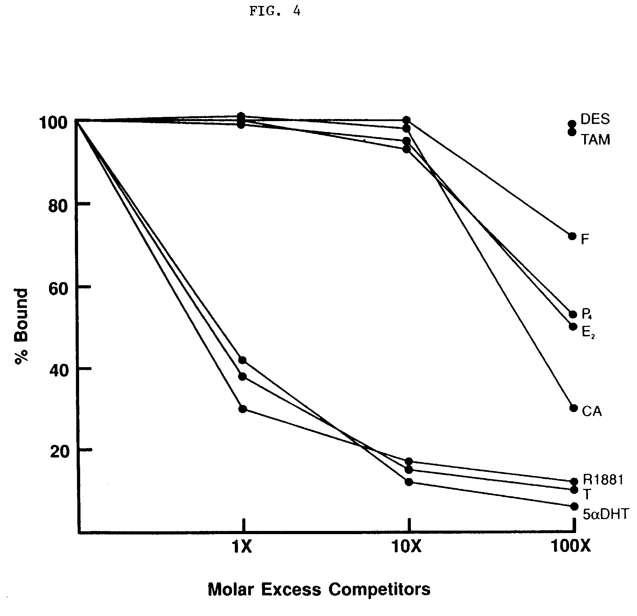

**FIGURE 4: Steroid Binding Specificity Of The Mouse Androgen Receptors**

Aliquots of yeast extracts containing the wild type androgen receptors are incubated with 10 nM [$^3$H]5$\alpha$-Dihydrotestosterone in the absence (100% bound) or presence of a 1-, 10- or 100 fold molar excess of radioinert 5$\alpha$ -Dihydrotestosterone (5$\alpha$DHT), testosterone (T), methyltrienolone (R1881), cyproterone acetate (CA), estradiol (E2), progesterone (P4), flutamide (F), tamoxfen (TAM) or diethylstibestrol (DES). Bound [$^3$H]-5$\alpha$ -Dihydrotestosterone is determined as described in the examples. Values represent the average of two separate experiments.

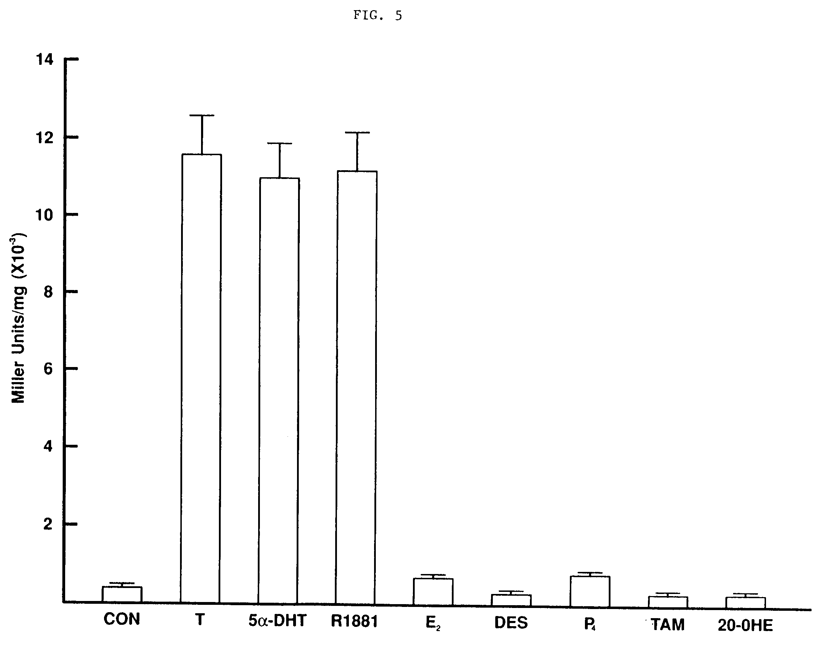

**FIGURE 5: Androgen-dependent Transcriptional Activation Of Receptors Expressed In Yeast Cells**

Yeast cells with the receptor expression and the reporter plasmids (YEpmAr/YRpA2) are grown in medium containing 10nM each testosterone (T), 5$\alpha$ -Dihydrotestosterone (5$\alpha$DHT), methyltrienolone (R1881), estradiol (E2), diethylstibestrol (DES), progesterone (P4), tamoxifen (TAM), 20-hydroxyecdysone (20-OHE) or no hormone (CON). Induction of $\beta$-galactosidase activity (Miller Units/mg protein) is measured in yeast extracts. Values represent three separate experiments with standard errors indicated.

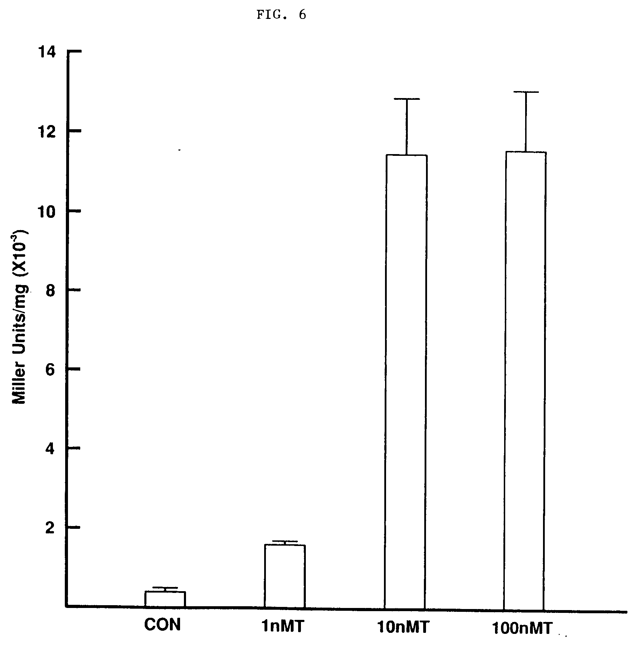

**FIGURE 6: Dose-dependence Of The Androgen Responsive Transcriptional Unit in Yeast**

Yeast cells (YEpmAR/YRpA2) are grown in medium containing no hormone, control (CON) or an increasing concentration of testosterone (1, 10 or 100 nM). The $\beta$-galactosidase activity (Miller Units/mg) is measured in yeast extracts. Values represent three separate experiments with standard errors indicated.

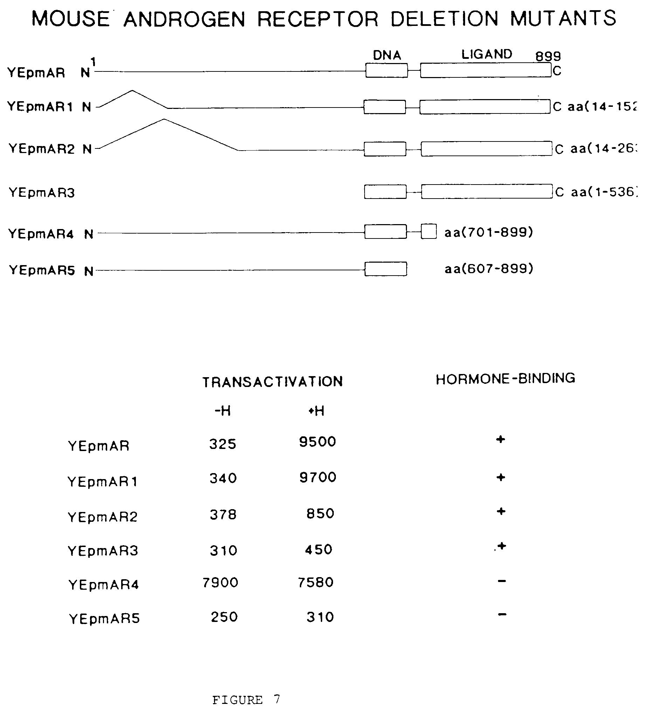

**FIGURE 7: Hormone Responsiveness Of Mouse Androgen Receptor Deletion Mutants Expressed In Yeast Cells**

(Top Panel) Schematic of wild type mouse androgen receptor deletion mutant (YEpmAR1, YEpmAR2, YEpmAR3, YEpmAR4 and YEpmAR5). The amino acid numbers (aa) are the deleted regions as described in the examples. (Botton Panel). Transcriptional activity of the mouse androgen receptor deletion mutants. Yeast cells with the receptor expression and the reporter plasmids are grown in medium in the absence (empty bar) or presence (hatch bar) of 10 nM of testosterone. The induction of $\beta$-galactosidase activity (Miller Units/mg) is measured in yeast extracts. Values represent the mean of two separate experiments. Hormone binding activities of the wild type receptor and receptor deletion mutants are analyzed as per the examples. However, specific binding is not detected for YEpmAR4 and YEpmAR5.

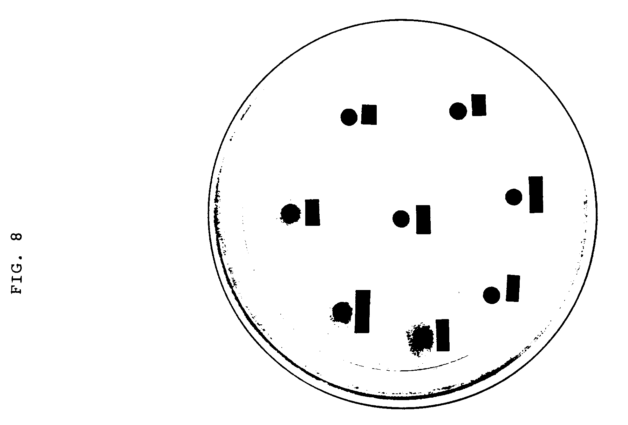

**FIGURE 8: Development Of A Plate Assay As a Screen To Detect Androgen Receptor Agonists**

Yeast cells (YEpmAR/YRpA2) containing the receptor expression and the reporter plasmids are grown in SD liquid medium overnight at 30ºC. These cells (2%) are inoculated on yeast agar plates containing the chromogenic substrate, 5-bromo-4-chloro-4-indolyl-B-D-galactoside (X-gal, 40 ug/ml). Discs containing 5 ng each testosterone (T), 5$\alpha$-Dihydrotestosterone (5$\alpha$DHT), methyltrienolone (R1881), estradiol (E2), progesterone (P4), tamoxifen (TAM), 20-hydroxyecdysone (20-OHE) or diethylstibestrol (DES) are placed on top of the agar. After overnight incubation at 30ºC, induction of $\beta$-galactosidase activity is measured by the formation of a blue halo around the test compound.

**SUMMARY OF THE INVENTION**

The present invention relates to a novel androgen receptor screening method. The purpose of this method is to study and/or identify compounds which act as agonist and/or antagonist of hormones such as the androgen hormones.

EP 0 577 932 A2

Further, the present invention also relates to mutant androgen receptor mutants and identification of the transcriptional activation domains of the mouse androgen receptor.

Specifically, a yeast-based expression system is used in the present invention thereby enhancing the ease in which screening is accomplished. Expression of a fusion protein of the mouse androgen receptor and a yeast ubiquitin fusion system is under the control of a constitutive yeast promoter, triose phosphate dehydrogenase (TDH3). A polypeptide with the predicted size of the mouse androgen receptor (98 kDa) is detected by Western immunoblots in yeast extracts using a polyclonal antibody directed against the N-terminal region of the human androgen receptor. The receptors expressed in yeast cells exhibit hormone binding affinity and specificity characteristics of the authentic mouse androgen receptor. Hormone- dependent transcriptional activation of the expressed receptors is studied by transforming yeast cells with a reporter plasmid that contains two copies of a putative androgen response element (a GRE/PRE of the glucocorticoid responsive tyrosine aminotransferase gene) upstream of the yeast proximal CYC1 promoter fused to the lac-Z gene of E. Coli. The induction of $\beta$-galactosidase is strictly dependent on natural and synthetic androgens as well as the presense of a putative androgen response element.

The level of androgen receptor expression is ten fold lower than those reported for estrogen, glucocorticoid and progesterone receptors expressed in yeast cells. Although these observations might be due to different yeast promoters, a similar level of receptor expression (100-120 fmol/mg protein) is observed when the mouse androgen receptor is expressed in insect cells using a baculovirus expression system. The biochemical characteristics of the receptors expressed in yeast cells are similar to those reported for the authentic mouse androgen receptor (Kd = 0.9 nM). Although steroid binding competition analyses indicate that natural and synthetic androgens are excellent competitors, high concentrations of progesterone or estrogen can also be competing for the tritiated dihydrotestosterone binding sites, correlating with the lac-Z gene induction by these steroids at high concentrations. Antiandrogens, cyproterone acetate and hyroxyl-flutamide can also be competing for the androgen receptor binding sites at high concentrations indicating that these compounds have lower affinity for the androgen receptor.

It is an object of the present invention, therefore, to provide a simple, economically attractive method to screen for hormone agonists and/or antagonists. It is another object of the present invention to identify the critical transcriptional activity centers of the androgen receptor. Two such regions are amino acid sequences 152-263 at the N-terminus of the deposited receptor and amino acid 607-701. By utilizing the method of the present invention, derivatives of the receptor may be developed to produce new therapeutic compounds. These and further objects of the invention are disclosed in the detailed description of the invention provided hereinbelow.

DETAILED DESCRIPTION OF THE INVENTION

Although previously reported that the chicken progesterone receptor and the human Vitamin D3 receptor can be functionally expressed in yeast, Saccharomyes cerevisiae (13), the present invention provides for the expression of full length mouse androgen receptor and a series of deletion mutants in yeast cells. The expressed receptors are analyzed by ligand binding and Western immunoblots.

Hormone-dependent transcriptional activation is studies by co-transformation of wild type or mutant receptors with a reporter plasmid in yeast cells. The results of these studies demonstrate the usefulness of using a simple eukaryotic system such as yeast to dissect the functional domains of the mouse androgen receptor, specifically, the N-terminal domain involved in transactivation and the carboxyl-terminal hormone binding domain. The expression system described permits the development of agonists, although antagonists are exemplified.

To study the structure-function relationships of the mouse androgen receptor, a series of receptor mutants are constructed and used to transform yeast cells containing the reporter plasmids. Expressed receptor proteins from these mutants are analyzed by hormone binding, immunoblots and transcriptional activation. N-terminal deletion analyses indicate that the amino-terminus of the receptor is not required for hormone binding. However, transcriptional analyses reveal an internal region within the N-terminus essential for full transcriptional activity. Deletion of the entire ligand binding domain results in a potent constitutive transactivator. The present invention demonstrates the usefulness of a "simple" eukaryotic system to detect the functional domains of ligand-activated transcription factors. Comparative analyses of the androgen receptor functional domains in yeast and mammalian cells facilitate the identification of new transcription factors involved in the pathway of androgen action.

The following examples are provided to describe the present invention and are not limitative of it.

The restriction enzymes, T4 polynucleotide kinase and T4 DNA ligase used in the following examples are purchased from New England Biolabs. Calf intestinal alkaline phosphatase is purchased from United

4

States Biochemical Corporation. Taq polymerase is bought from Perkin-Elmer Cetus. 1,2,5,6,7-[$^3$H]5-$\alpha$ Dihydrotestosterone(120.3 Ci/mmol) is purchased from DuPont (New England Nuclear). Radioinert steroids, cyproterone acetate adenine sulfate, o-nitrophenyl $\beta$-D-galactopyranoside and uracil are acquired from Sigma. Casamino acids, yeast nitrogen base and dextrose are purchased from Difco. Glassperlen is bought from B.Braun Melsungen AG. The alkaline phosphatase conjugate immunoblot assay kit is purchased from BioRad.

For ease in reviewing the examples, the following is provided. Homogenization buffer used contains 10mM Tris, 0.1 mM EDTA, 2mM dithiothreitol and 10% glycerol, pH 8.1 (TEDG buffer). Transcription buffer for $\beta$-galactosidase assay contains 0.12 M sodium phosphate (dibasic), 0.04 M sodium phosphate (monobasic), 10 mM potassium chloride, 1 mM magnesium sulfate and 0.27% 2-mercaptoethanol, pH 7.0.

Finally, the Saccharomyces cerevisiae strain used in the present invention is BJ3505 (Mata pep4::his3 PR61-$\Delta$1.6R his3lys2-208trp1-$\Delta$101 ura3-52 gal2 (17) obtained from that source. Expression of the mouse androgen receptor is under the control of a constitutive yeast promoter, triose phosphate dehydrogenase (TDH3). Growth and transformation of yeast cells is performed according to standard procedures (18).

Briefly, yeast cells are grown in 10 ml of YEPD medium overnight at 30ºC until cell density reachs and O.D. of 1.0. Cells are then collected by centrifugation and washed with 10 ml of 0.1M lithium acetate. Cells are then resuspended in 10 ml 0.1M lithium acetate and incubated at 30 ºC for 1 hour. Cells are collected and resuspended in 0.5 ml 0.1M lithium acetate. Aliquots of cells (50 $\mu$l) are incubated with 1-2$\mu$g DNA and incubated for 10 min at 30ºC followed by 1 hour incubation in the presence of 0.5 ml 40% polyethyleneglycol (4000). The cell mixture is heated for 5 min at 42ºC. Cells are then collected and plated onto appropriate yeast media. Transformants are selected by tryptophan auxotrophy.

The plasmids, DNA sequences and microorganisms deposited in connection with the present patent application, except where specified to the contrary, are deposited in American Cyanamid Company's culture collection maintained in Princeton, New Jersey and are deposited pursuant to the Budapest Treaty with the American Type Culture Collection (ATCC) in Rockville, Maryland 20952, U.S.A.

The following E. coli bacterial strains were deposited in the ATCC on March 12, 1992. These include YEpmAR$_1$, (ATCC 68925), YEpmAR$_2$ (ATCC 68926), YEpmAR$_4$ (ATCC 68927) and YEpmAR$_5$ (ATCC 68928).

The following Saccharomyces cerevisiae yeast strains were deposited in the ATCC on March 12, 1992. These include BJYEpmAR (ATCC 74137), BJYEpmAR$_1$ (ATCC 74138), BJYEpmAR$_2$ (ATCC 74139), BJYEpmAR$_4$ (ATCC 74140), BJYEpmAR$_5$ (ATCC 74141) and BJYRpA$_2$ (ATCC 74142).

## EXAMPLE 1

### Construction of Expression Vectors

The full length mouse androgen receptor coding sequence is cloned into the BamH1 site of the eukaryotic expression vector (pcDNA/neomycin). This plasmid DNA is digested to completion with DraIII to obtain the entire coding sequence of the androgen receptor plus an additional 830 base pairs upstream of the initiation codon. This resulting fragment is subsequently digested with Afl II to obtain a 2.23 kb fragment which encodes the truncated androgen receptor with a deletion of 152 amino acids from the N-terminus. A linker containing a unique BssHII site is ligated to the DraIII site after the stop codon. Oligonucleotide primers are synthesized with an Applied Biosystems 380A DNA synthesizer. Using PCR-mediated mutagenesis, a unique EagI site is created near the initiation condon by changing the proline (CCC) at position 14 to proline (CCG). The resulting PCR fragment (417 base pairs) contains a new EagI site at the 5' end and an Af1 II site at the 3' end. In order to produce an in frame ubiquitin-fusion receptor protein under the control of a constitutive yeast promoter (TDH3), an oligonucleotide encoding the last six amino acids of the ubiquitin gene fused to the amino terminus of the mouse androgen receptor (36 base pairs downstream from the initiation codon followed by an internal EagI and BssHII sites) is synthesized. This oligonucleotide is cloned into the Afl II-KpnI sites of the high copy number yeast expression vector (Fig 1 A). This resulting expression plasmid (YEpV5) is digested to completion with EagI-BssHII and ligated to the 417 base pairs PCR fragment and the 2.23 kb truncated receptor. The resulting receptor expression vector (YEpmAR) (Fig 1A) is used to transform Trp1-deleted S. cerevisiae strain BJ3505 using the lithium acetate protocol. Transformants are selected by tryptophan auxotrophy.

## EXAMPLE 2

### Construction of Reporter Plasmid

An oligonucleotide containing two copies of a putative androgen response element (a GRE/PRE of the glucocorticoid responsive tyrosine aminotransferase gene)(15) is cloned into the unique XhoI site of the yeast reporter plasmid YRpPC2(13). The nucleotide sequence of the double-stranded GRE/PRE is as follows:

$$\text{GATCCTGTACAGGATGTTCTAGCTACG}$$

$$\text{GACATGTCCTACAAGATCGATGCCTAG}$$

The resulting reporter plasmid (YRpA2) (Fig 1B) contains the putative androgen response element (to -250 base pairs) of the iso-1-cytochrome c (CYC1) promoter which is fused directly to the $\beta$-galactosidase gene of E. coli. These plasmids are used to tranform yeast cells expressing the mouse androgen receptor (YEpmAR). Transformants are subsequently selected by tryptophan and uracil auxotrophy.

## EXAMPLE 3

### Construction of Mutant Receptors

The entire coding sequence of the mouse androgen receptor is excised from the eukaryotic expression vector (mAR/pcDNA1/neo) and inserted into the BamH1 site of psP72. A unique BssH II site is cloned into the Dra II site after the stop codon. The plasmid DNA is digested with Afl II-BssHII and Afl III-BssHII to generate the 2.23 kb and 1.92 kb fragments which encode two truncated androgen receptors with internal deletions of 139 and 250 amino acids from the N-terminus. After ligating an EagI site at the 5' end, these fragments are inserted into the yeast expression vector (YEpV5). The resulting constructs are called YEpmAR$_1$ and YEpmAR$_2$ (Fig 7). A truncated receptor containing only the DNA and ligand-binding domain is constructed by PCR amplification resulting in a 1.12 kb fragment flanked by EagI and BssHII sites. This PCR fragment is then inserted into the yeast expression vector leading to YEpmAR$_3$. A mutant receptor (YEpmAR$_4$) with a deletion of 200 amino acids from the carboxyl-terminus is made by digesting the plasmid DNA with StuI-BssHII and religated in the presence of a linker with an in frame stop codon. The resulting plasmid is digested with Afl II-BssHII to obtain a 1.64 kb fragment that is ligated to the yeast expression in the presence of the 417 base-pairs PCR fragment described previously. The last C-terminal deletion mutant is constructed by PCR amplification and cloned into the yeast expression vector leading to YEpmAR$_5$. All PCR-generated fragments and mutant receptors are sequenced to confirm proper reading frame.

## EXAMPLE 4

### Preparation of Yeast Extracts

Yeast cells containing the full length receptor (YEpmAR) or deletion mutant receptors are grown overnight at 30°C in medium containing 2% dextrose, 0.1% casamino acids, 0.67% yeast nitrogen base, 0.001% adenine and 0.002% uracil. When cell density reaches mid-log phase, cells are harvested and washed with receptor buffer (TEDG). This and all subsequent steps are carried out at 4°C. The final cell pellets are suspended in TEDG buffer and mixed with an equal volume of glass beads. Cells are disrupted by vortexing. The homogenate is centrifuged at 1000x g for 10 min and the supernatant further centrifuged at 100,000 x g for 30 min to obtain yeast extracts which are used immediately or stored at -70°C for subsequent analysis. Protein concentration is determined by RAD protein assay according to manufacturer's protocol using bovine serum albumin as standard. Briefly, dye reagent concentrate is dilute (1:5) with distilled water. 2-4 $\mu$l of samples are added to one ml of the diluted dye solution and incubated for 5 min at room temperature. O.D. values are taken at wavelength of 595 against the blank.

### EXAMPLE 5

**Hormone Binding Assays**

Yeast extracts containing 500-800 $\mu$g protein are incubated overnight with 10 nM $[^3H]5\alpha$-Dihydrotestosterone in the absence (total binding) and the presence (non-specific binding) of a 100-fold excess of radioinert $5\alpha$-Dihydrotestosterone. Bound and free steroids are separated by dextran-coated charcoal (0.5% Norit A and 0.05% dextran). Specific binding is determined by subtracting non-specific from total binding. Scatchard analyses and relative binding affinity of the receptor are determined to those skilled in the art as exemplified in (17,18).

Briefly, yeast extracts are labeled overnight at 4°C with an increasing concentration of $[^3H]5\alpha$-Dihydrotestosterone (0.5-7.0 nM) in the absence or presence of a 100-fold molar excess of radioinert $5\alpha$-Dihydrotestosterone. Specific binding is calculated from the saturation curve and converted into a Scatchard plot. The Kd and the Bmax are obtained from the scope and the Y intercept of the plot.

### EXAMPLE 6

**Western Immunoblot Analysis**

Yeast extracts (100 $\mu$g protein) are resolved on a 7.5% SDS-polyacrylamide gel. A polyclonal anti-androgen receptor antibody (19) is used as a first antibody to probe the mouse androgen receptor. The second antibody is goat anti-rabbit (IgG) conjugated with alkaline phosphatase as described by Bio-Rad Laboratories.

In this procedure, the membrane is washed twice after incubating with the second antibody and subsequently immersed in the freshly prepared alkaline phosphatase color developing buffer. Immunoreactive proteins are observed within 5 minutes as distinct purple bands on the membrane.

### EXAMPLE 7

**Transcription Assays**

Yeast strains containing the receptor and the reporter plasmids (YEpmAR/YRpA$_2$) are grown in minimal medium (without tryptophan and uracil) in the absence or presence of 100 nM testosterone or other hormones. When cell density reaches late-log phase, yeast extracts are prepared from the cells and assayed for $\beta$-galactosidase activity (13). Enzyme activity is expressed as Miller units/mg protein (20).

In this assay, aliquots of yeast extracts (50-100$\mu$g) are incubated with the substrate, ONPG(o-nitrophenyl-$\beta$-D-Galactopyranoside) at 28°C for one hour. The reaction is stopped by addition of 1M sodium carbonate. O.D. values are taken at the wavelength of 420. The enzyme activity is expressed as Miller units/mg protein and is calculated according to Miller (20) as follows:

Enzyme activity = OD(420) X 1000/TxP where T is the time of incubation in minutes and P is the amount of protein in mg.

### EXAMPLE 8

**Biochemical Analysis Of Receptors Expressed In Yeast Cells**

Yeast extracts containing wild type and deletion mutant receptors are analyzed by Western immunoblots. As shown in Fig 2, a distinct polypeptide with the predicted size of the full length mouse androgen receptor (M.W. = 98 kD) is detected in yeast extracts containing the wild type mouse androgen receptor. The two N-terminal deletion mutants (YEpmAR$_1$ and YEpmAR$_2$) are also detected by the same antibody with apparent molecular weights of 80 kD and 66 kD respectively (Fig 2). The deletion mutant (YEpmAR$_3$) that lacks the entire N-terminal regions is not detected by this antibody. The predicted size of these expressed receptor proteins indicates that the ubiquitin is cleaved off the fusion protein by the yeast host enzyme (13). The full length receptors expressed in yeast cells are analyzed for hormone binding characteristics. As shown in Fig 3A, specific binding of the receptors approaches saturation at 3-5 nM$[^3H]$ $5\alpha$-Dihydrostestosterone. Scatchard analysis of this data indicate a single class of androgen binding components with an apparent Kd of 0.96 nM and a maximum binding capacity of 110 fmol/mg protein (Fig 3B). The binding specificity of these receptors is also examined by labeling the extracts with 10 nM $[^3H]5\alpha$-

Dihydrotestosterone in the presence or absence of 1, 10 and 100 fold molar excess of radioinert competitors. As shown in Fig 4, a 10 fold excess of either natural androgens (testosterone or $5\alpha$-Dihydrotestosterone) or a synthetic androgen (R1881) compete effectively (85-88%), whereas other steroid hormones and antihormones are poor competitors. However, 100 fold molar excess of estrogen or progesterone displaces 50% of the total binding. The binding activities of the deletion mutants are also analyzed and compared to that of the full length receptor. All deletion mutant receptors except YEpmAR$_4$ and YEpmAR$_5$ with deletions in the ligand binding domain are able to bind [$^3$H]$5\alpha$-dihydrotestosterone (Table 1).

TABLE I

| CONSTRUCT | AR BINDING (fmol/mg) |
|---|---|
| YEpmAR | 98 |
| YEpmAR1 | 75 |
| YEpmAR2 | 80 |
| YEpmAR3 | 45 |
| YEpmAR4 | Not detected |
| YEpmAR5 | Not detected |

Yeast extracts containing the wild type receptor or the receptor deletion mutants are labelled with 10 nM [$^3$H]5$\alpha$-Dihydrotestosterone in the absence or presence of 100-fold molar excess of radioinert 5 $\alpha$-Dihydrotestosterone. Androgen receptor (AR) binding is determined as described. Values represent the average of two separte experiments.

**EXAMPLE 9**

**Functional Analysis Of The Mouse Androgen Receptors Expressed In Yeast**

The transcriptional activity of the mouse androgen receptors produced in yeast cells is studied by transforming the yeast cells (YEpmAR) with a reporter plasmid containing two copies of GRE/PRE of the tyrosine aminotransferase gene upstream of the yeast promoter, iso-1-cytochrome c which is fused to the lac-Z gene of E. coli (Fig 1 B). The induction of the lac-Z gene is strictly dependent on specific ligands and the presence of a putative androgen response element. Ten nM of natural or synthetic androgens induce the $\beta$-galactosidase activity by 28-30 fold whereas other steroid hormones are not effective to induce the reporter gene (Fig 5). However, estrogen or progesterone at higher concentration (1 $\mu$M) also induce the lac-Z gene by 12-15 fold. The androgen dependent induction of the lac-Z gene is abolished when the two copies of GRE/PRE are replaced by the estrogen response element (ERE)(21). The transcriptional activity of the mouse androgen receptors also depends on the concentration of androgen present in the culture media. Three to four fold induction of $\beta$-galactosidase activity is observed by 1 nM of testosterone. Maximal induction of the reporter enzyme is achieved by 10 nM of the hormone (Fig 6).

**EXAMPLE 10**

**Functional Analysis Of Receptor Deletion Mutants**

The transcriptional activities of all the receptor deletion mutants are shown in Fig 7. Deletion of the entire amino-terminus (YEpmAR$_3$) results in complete loss of androgen dependent induction of transcriptional activity. Deletion of 250 amino acids from the N-terminus (YEpmAR$_2$) shows only 2 to 3 fold induction in transcriptional activity whereas the mutant, YEpmAR1 with a deletion of 139 amino acids from the N-terminus, has a similar fold of induction as the wild type receptor. The mutant (YEpmAR$_4$) with a gross deletion in the ligand binding domain gives high constitutive transcriptional activity comparable to that of the wild type receptor. The last mutant (YEpmAR$_5$) with deletions in the entire ligand binding domain and the hinge region has no transcriptional activity. The transcriptional analysis data indicate that the mouse androgen receptors function in yeast cells. The induction of the lac-Z gene is dependent on natural and synthetic androgens, the presence of androgen receptors and a steroid response element (GRE/PRE). It has been well documented that the glucocorticoid response element (GRE/PRE) derived from mouse mammary tumor virus-long terminal repeat (MMTV-LTR) mediates androgen responsiveness of a NMTV-CAT reporter gene in mammalian cell transfection studies. This androgen response element (ARE) is supported by the fact that two copies of the GRE/PRE of the glucocorticoid responsive tyrosine aminotransferase gene are used as cis-acting elements. It is interesting to note that the lac-Z gene could be induced when high concentration (1 $\mu$M) of cyproterone acetate is tested in this hormone responsive transcription unit. Similar results are observed when this antihormone is tested in mammalian cell transfection studies indicating that this compound can be an agonist at high concentrations. In the present invention, induction of the reporter enzyme, $\beta$-galactosidase, could be detected by 1 nM of testosterone. This has resulted in a plate assay invention in which the induction of the reporter enzyme, as measured by the formation of a blue zone (when X-gal is used as a chromogenic substrates), is detected by less than 1 ng of testosterone (Fig 8). Thus this hormone responsive transcription unit is used as a highly sensitive screen to detect novel androgen receptor agonists. Similarly, antagonists with partial agonist activity can also be detected.

**EXAMPLE 11**

**Functional Domains Of Androgen Receptor**

The yeast expression system of the present invention also provides a mechanism or method of analyses for the functional domains of the androgen receptor. As predicted from studies on the human androgen receptor and other steroid receptor superfamily (6,23), the amino-terminus of the mouse androgen receptor is not required for hormone binding as reflected by the ability of all the N-terminal deletion mutants to bind androgens. However, transcriptional activation is dramatically reduced with an internal deletion (amino acids 152-263) whereas the mutant with an internal deletion (amino acids 14-152) is able to transactivate the reporter gene like the wild type. This observation strongly suggests that the internal region (amino acids 152-263) at the N-terminus of the mouse androgen receptor is essential for transcriptional activity. Removal of the entire N-terminal domain results in complete loss of gene activation indicating another region downstream (amino acid 263-536) is also important for transcriptional activity. There is compelling evidence that the N-terminal domains of the chicken progesterone receptor and glucocorticoid receptor play a key role in target gene specificity and transactivation (24,25). More recently, N-terminal deletion analyses in mammalian cell transfection studies also indicate that a region of the human androgen receptor (amino acids 141-338) contains a signal sequence essential for transcriptional activity. Interestingly, both internal deleted regions of the mouse (amino acid 152-263) and the human (amino acids 141-338) androgen receptors contain a long polyglutamine stretch, which is also present in the rat androgen receptor (2,3). Although the mechanisms involving transcriptional activation by these internal sequences at the N-terminal domain are not known, it is highly suggestive that this region is involved in protein-protein interactions. This is further supported by the observation that the transcriptional activation of the human androgen receptor is inhibited by coexpression of the amino-terminal domain of the androgen receptor. Also, patients suffering from spinal and bulbar muscular atrophy have a defect in the androgen receptor gene that is characterized by an enlargement of the glutamine residues present in the N-terminus (12). Many transcription factors such as Sp1 (26), TF11D (27) and GCN4 (28) contain long polyglutamine stretches suggesting these glutamine residues may be involved in transcriptional regulation by interacting with specific nuclear factors. The demonstration of these similar essential transactivation regions found in the mouse and human androgen receptors using such phylogenically diverse backgrounds as yeast and mammalian cells strongly suggests that there may be common nuclear factor(s) involved in this transactivatonal pathway. It is therefore now possible to identify these factors using the method of the present invention .

It has been shown that removal of the hormone binding domains of the most steroid hormone receptors leads to constitutive active molecules with about 10% of transcriptional activity of the wild type receptors (29). In the present invention, the truncated mouse androgen receptor without the hormone binding domain is also constitutive active in yeast cells. However, ligand independent transcriptional activity of these deletion mutants is much higher (60-70% of the wild type) indicating that they are potent transactivators in yeast cells. Unliganded steroid receptors (aporeceptors) are associated with 90k heat shock protein (hsp90) and it has been suggested that the ligand binding domain represses transcriptional activation of steroid receptors in the absence of hormone (30,31). The results observed with the present invention are in agreement with this model.

Comparative mutational analyses of the androgen receptor in yeast cells versus mammalian cells are useful to facilitate the identification of new transcription factors which participate in the pathway of androgen receptor function.

For clarity purposes, the sequence listing portions contain amino acids 152-263 (identified as 1-110 in the first sequence listing) and amino acids 607-701 (identified as 1-48) in the second sequence listing).

**Bibliography**

1. Fang, S., Anderson, K.M. and Liao, S. (1969). J. Biol.Chem244, 6584-6588.
2. Lubuhn, D.B., Joseph, D.R., Sullivan, P.M., Willard, H.F., French, F.S. and Wilson, E.M. (1988). Science 240,327-330.
3. Chang, D., Kokontis, J. and Liao, S. (1988). Science 240,324-326.
4. Trapman, J., Klaassen, P., Kuiper G.G.J.M., van der Korput, J.A.G.M., Faber, P.W., van Rooij, J.C.J., Geurts van Kessel, A., Voorhorst, M.M., Mulder, E. and Brinkmann, A.O. (1988). Biochem. Biphys.Res. Commun. 153,241-248.
5. Tilley, W.D., Masrcelli, M., Wilson, J.D. and McPhaul, M.J.(1989). Proc. Natl. Acad. Sci.(USA).86, 327-331.
6. Evans, R.M. (1988).Science 13, 889-895.
7. O'Malley, B.W. (1990).Mol.Endocrinol.4,363-369.
8. Yamamoto, K.R. (1985).Annu.Rev.Genet. 19,209-252.
9. Beato, M. (1989).Cell 56,335-344.
10. Charest, N.J., Zhou, Z-X., lubahn, D.B., Olsen, K.L., Wilson, E.M. and French, F.S. (1991). Mol. Endocrinol. 5,573-581.
11. Faber, P.W., King, A., van Rooij, H.C.J., Brinkmann, A.O., de Both, N.J. and Trapman, J. (1991). Biochem. J. 278, 269-278.
12. La Spada, A.R., Wilson, E.M., Lubahn, D.B., Harding, A.E. and Fischbeck, K.H. (1991). Nature 352,77-79.

13. McDonnell, D.P., Pike, J.W., Drutz, D.J., Butt, T.R. and O'Malley, B.W. (1989). Mol. Cell. Biol. 9,3517-3523.

14. Moenle, C.M., Ayanardi, M.W., Kolodny, M.R., Park, F.J. and Jones, E.W. (1986). Genetics 115,255-265.

15. Bagchi, M.K., Elliston, J.F.,Tsai, S.Y., Edwards, D.P., Tsai, M-J. and O'Malley, B.W. (1988).Mol. Endocrinol.2,1221-1229.

16. Bradford, M.M. (1976). Anal. Biochem. 72,248-254.

17. Mak, P. and Callard, G.V. (1985). Characterization of estrogen receptors in the hamster brain. J. Steriod Biochem. 22,355-361.

18. Callard, G.V. and Mak, P. (1985). Exclusive nuclear location of estrogen receptors in Squalus testis. Proc. Natl. Aca. Sci(USA). 82, 1336-1340.

19. Young, CY-F., Murthy, L.R., Prescott, J.L., Johnson, M.P., Rowley, D.R., Cunningham, G.R., Killian, C.S., Scardino, P.T., von Eschenbach A. and Tindall, D.J.(1988).Endocrinol. 123,601-610.

20. Miller, J.H. (1972) in Experiments in Molecular Genetics (Miller, J.H., ed)pp.352-355, Cold Spring Harbor Laboratory, Cold Spring Harbor, NY.

21. Klein-Hitpass, L., Schorpp, M., Wagner, U. and Ryffel, G.U. (1986). Cell 41,1055-1061.

22. Privalsky, M.L., Sharif, M. and Yamamoto, K.R. (1990). Cell 63,1277-1286.

23. Dobson, A.D.W., Conneely, O.M. Beattie, W., Maxwell, B.L., Mak P., Tsai, M-J., Schrader, W.T. and O'Malley, B.W. (1989). J. Biol. Chem. 264,4207-4211.

24. Tora, L., Gronemeyer, H., Turcotte, B., Gaub, M.P. and Chambon, P. (1988).Nature 33,185-188.

25. Godowski, P.J., Picard, D. and Yamamoto, K.R. (1988). Science 241,812-816.

26. Courey, A., Holtzman, D.A., Jackson, S.P. and Tjian, R. (1989). Cell 59,827-836.

27. Peterson, M.G., Tanese, N., Pugh, B.F. and Tjian, R. (1990). Science 248,1625-1630.

28. Hope, I. and Struhl, K. (1986). Cell 46,885.

29. Carson, M.A., Tsai, M-J., Conneely, O.M., Maxwell, B.L., Clark, J.H. Dobson, A.D.W., Elbrecht, A., Toft, D.O., Schrader, W.T. and O'Malley, B.W. (1987). Mol. Endocrinol. 11,791-801.

30. Picard, D., Khursheed, B., Garabedian M.J., Fortin, M.G., Lindquist, S. and Yamamoto, K.R. (1990). Nature 348, 166-169.

31. Groyer, A., Schweizer-Groyer, G., Cadepond, F., Mariller, M. and Baulieu, E.E. (1987). Nature 328,624-628.

SEQUENCE LISTING

(1) GENERAL INFORMATION:

(i) APPLICANT:  Paul Mak

(ii) TITLE OF INVENTION:   Development of a Mechanism-based Screen To Identify Androgen Receptor Agonists and Antagonists

(iii) NUMBER OF SEQUENCES:   2

(iv) CORRESPONDENCE ADDRESS:

(A) ADDRESSEE:   Dr. Estelle J. Tsevdos, American Cyanamid Company

(B) STREET:   1937 West Main Street, P. O. Box 60

(C) CITY:   Stamford

(D) STATE:   Connecticut

(E) COUNTRY:   United States Of America

(F) ZIP:   06904-0060

(v) COMPUTER READABLE FORM:

(A) MEDIUM TYPE:   Floppy Disk

(B) COMPUTER:   IBM PC AT

(C) OPERATING SYSTEM:   MS-DOS

13

(D) SOFTWARE:   ASCII converted from IBM Displaywrite 4

(vi) CURRENT APPLICATION DATA:

(A) APPLICATION NUMBER:

(B) FILING DATE:

(C) CLASSIFICATION:

(vii) PRIOR APPLICATION DATA:

(A) APPLICATION NUMBER:

(B) FILING DATE:

(viii) ATTORNEY/AGENT INFORMATION:

(A) NAME:   Tsevdos, Estelle J., Dr.

(B) REGISTRATION NUMBER:   31,145

(C) REFERENCE/DOCKET NUMBER:   31,274-00

(ix) TELECOMMUNICATION INFORMATION:

(A) TELEPHONE:   203 321 2756

(B) TELEFAX:   203 321 2971

(C) TELEX:   710 474 4059

(2) INFORMATION FOR SEQ ID NO: 1:

(i) SEQUENCE CHARACTERISTICS:

(A) LENGTH:   330 base pairs

(B) TYPE:   nucleic

(C) STRANDEDNESSS:   single

(D) TOPOLOGY:   linear

(ii) MOLECULE TYPE:   DNA

(iii) HYPOTHETICAL:

(iv) ANTI-SENSE:

(v) FRAGMENT TYPE:

(vi) ORIGINAL SOURCE:

(A) ORGANISM:

(B) STRAIN:

(C) INDIVIDUAL ISOLATE:

(D) DEVELOPMENTAL STAGE:

(E) HAPLOTYPE:

(F) TISSUE TYPE:

(G) CELL TYPE:

(H) CELL LINE:

(I) ORGANELLE:

(vii) IMMEDIATE SOURCE:

(A) LIBRARY:

(B) CLONE:

(viii) POSITION IN GENOME:

    (A) CHROMOSOME/SEGMENT:

    (B) MAP POSITION:

    (C) UNITS:

(ix) FEATURE:

    (A) NAME/KEY:

    (B) LOCATION:

    (C) IDENTIFICATION METHOD:

    (D) OTHER INFORMATION:

(x) PUBLICATION INFORMATION:

    (A) AUTHORS:

    (B) TITLE:

    (C) JOURNAL:

    (D) VOLUME:

    (E) ISSUE:

    (F) PAGES:

    (G) DATE:

(H) DOCUMENT NUMBER:

(I) FILING DATE:

(J) PUBLICATION DATE:

(K) RELEVANT RESIDUES:

(xi) SEQUENCE DESCRIPTION: SEQ ID NO: 1:

```
TTA AGC AGC TGC TCC GCC GAC ATT AAA GAC ATT TTG        36
Leu Ser Ser Cys Ser Ala Asp Ile Lys Asp Ile Leu
 1               5                   10

AAC GAG GCC GGC ACC ATG CAA CTT CTT CAG CAG CAG        72
Asn Glu Ala Gly Thr Met Gln Leu Leu Gln Gln Gln
         15                  20

CAA CAA CAG CAG CAG CAC CAA CAG CAG CAC CAA CAG        108
Gln Gln Gln Gln Gln His Gln Gln Gln His Gln Gln
 25                  30                  35

CAC CAA CAG CAG CAG GAG GTA ATC TCC GAA GGC AGC        144
His Gln Gln Gln Gln Glu Val Ile Ser Glu Gly Ser
         40                  45

AGC GCA AGA GCC AGG GAG GCC ACG GGG GCT CCC TCT        180
Ser Ala Arg Ala Arg Glu Ala Thr Gly Ala Pro Ser
         50                  55                  60

TCC TCC AAG GAT AGT TAC CTA GGG GGC AAT TCA ACC        216
Ser Ser Lys Asp Ser Tyr Leu Gly Gly Asn Ser Thr
             65                  70

ATA TCT GAC AGT GCC AAG GAG TTG TGT AAA GCA GTG        252
Ile Ser Asp Ser Ala Lys Glu Leu Cys Lys Ala Val
         75                  80
```

```
TCT GTG TCC ATG GGA TTG GGT GTG GAA GCA TTG GAA      288
Ser Val Ser Met Gly Leu Gly Val Glu Ala Leu Glu
 85                     90                  95

CAT CTG AGT CCA GGG GAA CAG CTT CGG GGA GAC TGC      324
His Leu Ser Pro Gly Glu Gln Leu Arg Gly Asp Cys
            100                 105
ATG TAC                                               330
Met Tyr
    110
```

(2) INFORMATION FOR SEQ ID NO: 2:

   (i) SEQUENCE CHARACTERISTICS:

     (A) LENGTH:   147 base pairs

     (B) TYPE:   nucleic acid

     (C) STRANDEDNESSS:   single

     (D) TOPOLOGY:   linear

  (ii) MOLECULE TYPE:   DNA

  (iii) HYPOTHETICAL:

  (iv) ANTI-SENSE:

  (v) FRAGMENT TYPE:

   (vi) ORIGINAL SOURCE:

     (A) ORGANISM:

     (B) STRAIN:

(C) INDIVIDUAL ISOLATE:

(D) DEVELOPMENTAL STAGE:

(E) HAPLOTYPE:

(F) TISSUE TYPE:

(G) CELL TYPE:

(H) CELL LINE:

(I) ORGANELLE:

(vii) IMMEDIATE SOURCE:

(A) LIBRARY:

(B) CLONE:

(viii) POSITION IN GENOME:

(A) CHROMOSOME/SEGMENT:

(B) MAP POSITION:

(C) UNITS:

(ix) FEATURE:

(A) NAME/KEY:

(B) LOCATION:

(C) IDENTIFICATION METHOD:

(D) OTHER INFORMATION:

(x)  PUBLICATION INFORMATION:

   (A)  AUTHORS:

   (B)  TITLE:

   (C)  JOURNAL:

   (D)  VOLUME:

   (E)  ISSUE:

   (F)  PAGES:

   (G)  DATE:

   (H)  DOCUMENT NUMBER:

   (I)  FILING DATE:

   (J)  PUBLICATION DATE:

   (K)  RELEVANT RESIDUES:

(xi)  SEQUENCE DESCRIPTION: SEQ ID NO: 2:

```
GGA GCT CGT AAG CTG AAG AAA CTT GGA AAT CTA AAA        36
Gly Ala Arg Lys Leu Lys Lys Leu Gly Asn Leu Lys
 1               5                   10

CTA CAG GAG GAA GGA GAA AAC TCC AAT GCT GGC AGC        72
Leu Gln Glu Glu Gly Glu Asn Ser Asn Ala Gly Ser
         15                  20

CCC ACT GAG GAC CCA TCC CAG AAG ATG ACT GTA TCA       108
Pro Thr Glu Asp Pro Ser Gln Lys Met Thr Val Ser
 25                  30                  35
```

```
CAC ATT GAA GGC TAT GAA TGT CAG CCT ATC TTT CTT       144
his Ile Glu Gly Tyr Glu Cys Gln Pro Ile Phe Leu
             40                        45


AAC                                                   147
Asn
   49
```

**Claims**

1. A method for screening androgen receptor agonists or antagonists, said method comprising: using a mouse androgen receptor expressed by a yeast expression system to screen antagonists of the steriod/thyroid receptor superfamily.

2. The method according to Claim 1, wherein said steriod/thyroid-based superfamily member is the androgen hormone receptor.

3. The method according to Claim 2, wherein said mouse androgen receptor is fused to the yeast ubiquitin fusion system.

4. The method according to Claim 3, wherein said yeast ubiquitin fusion system contains the yeast triosephosphate dehydrogenase (TDH3) promoter.

5. The method according to Claim 4, wherein said mouse androgen receptor system is contained in YEpmAR$_1$, YEmAR$_2$, YEmAR$_4$ or YEpmAR$_5$.

6. A method for determining the functional sites of the androgen receptor, said method comprising: expressing a series of mutant androgen receptors, expressed in a yeast expression system; analyzing the hormonal binding activity of antagonist or agonist binding to the expressed receptor; comparing such binding activity of the mutant receptors with known functional receptor regions; and determining the transcriptional activation domain of the mouse androgen receptor.

7. The method according to Claim 6, wherein said mutants are YEpmAR, YEpmAR$_1$, YEmpAR$_2$, YEpmAR$_3$, YEpmAR$_4$ or YEpmAR$_5$.

8. The method according to Claim 6, wherein said transcriptional activation domain is amino acids 152-263 of the mouse androgen receptor; or wherein said transcriptional activation doamin is amino acids 607-701 of the mouse androgen receptor.

9. YEpmAR$_1$

10. YEpmAR$_2$

11. YEpmAR$_4$

12. YEpmAR$_5$

13. The transcriptional activation domains of the androgen receptor.

14. The transcriptional activation domain according to Claim 13, which corresponds to amino acids 152-263 of the mouse androgen receptor or, which corresponds to amino acids 607-701 to the mouse androgen receptor.

FIG.1

(A)

(B)

EP 0 577 932 A2

FIG. 2

FIG. 3

EP 0 577 932 A2

FIG. 4

EP 0 577 932 A2

FIG. 5

FIG. 6

EP 0 577 932 A2

# MOUSE ANDROGEN RECEPTOR DELETION MUTANTS

| | TRANSACTIVATION | | HORMONE-BINDING |
|---|---|---|---|
| | -H | +H | |
| YEpmAR | 325 | 9500 | + |
| YEpmAR1 | 340 | 9700 | + |
| YEpmAR2 | 378 | 850 | + |
| YEpmAR3 | 310 | 450 | .+ |
| YEpmAR4 | 7900 | 7580 | − |
| YEpmAR5 | 250 | 310 | − |

FIGURE 7

FIG. 8